# EUROPÄISCHE PATENTSCHRIFT

(11) **EP 0 800 541 B1**
(45) Veröffentlichungstag und Bekanntmachung des Hinweises auf die Patenterteilung: **24.02.1999**
(21) Anmeldenummer: 95940935.0
(22) Anmeldetag: 27.12.1995
(51) Int. Cl.: C08F 283/12, C08L 21/00, C08L 9/00, C08G 77/442, G02B 1/04

(54) **SILOXANHALTIGE NETZWERKE**
SILOXANE-CONTAINING NETWORKS
RESEAUX CONTENANT DU SILOXANE

(30) Priorität: 30.12.1994 CH 3967/94; 30.12.1994 CH 3968/94
(43) Veröffentlichungstag der Anmeldung: 15.10.1997
(73) Patentinhaber: Novartis AG, 4058 Basel (CH)
(72) Erfinder: CHABRECEK, Peter, Clayton, VIC 3169 (AU); LOHMANN, Dieter, CH-4142 Münchenstein (CH)
(86) Internationale Anmeldenummer: CH9500308
(87) Internationale Veröffentlichungsnummer: WO9620964

(56) Entgegenhaltungen:
- EP-A- 0 279 414
- DE-A- 4 224 559
- FR-A- 2 641 785
- DATABASE WPI Week 8827 Derwent Publications Ltd., London, GB; AN 88-187368 XP002001802 & JP,A,63 125 541 (JAPAN SYNTHETIC RUBBER) , 28.Mai 1988
- DATABASE WPI Week 9145 Derwent Publications Ltd., London, GB; AN 91-328014 XP002001803 & JP,A,03 217 480 (GOYO SHIKO KK) , 25.September 1991
- DATABASE WPI Week 7737 Derwent Publications Ltd., London, GB; AN 77-66045y XP002001804 & JP,A,52 093 718 (SHINETSU CHEM IND KK) , 6.August 1977
- DATABASE WPI Week 8008 Derwent Publications Ltd., London, GB; AN 80-13926c XP002001805 & JP,A,55 005 904 (TOKYO SHIBAURA ELEC) , 17.Januar 1980
- DATABASE WPI Week 7434 Derwent Publications Ltd., London, GB; AN 74-60530v XP002001806 & JP,A,49 007 346 (SAKAI CHEM IND ) , 23.Januar 1974

## Beschreibung

Die vorliegende Erfindung beschreibt ein vernetztes Polymer, das aufgebaut ist aus einem Polymerisationsprodukt eines hoch ungesättigten Polymers (a) und eines Polysiloxans (b) mit terminalen oder pendenten Hydrosilangruppen, wobei noch zahlreiche ungesättigte Kohlenstoff-Kohlenstoffbindungen erhalten bleiben, auf welche gegebenenfalls mindestens ein vinylisches Monomer aufgepfropft ist; Verfahren zur Herstellung vernetzter Polymere; ophthalmische Formkörper, insbesondere Kontaktlinsen oder künstliche Hornhaut bestehend vollständig oder im wesentlichen aus solchen vernetzten Polymeren; ferner die Verwendung vernetzter Polymere für die Beschichtung von Oberflächen von ophthalmischen Formkörpern.

Polysiloxan-(Meth)acrylat Copolymere, die beispielsweise durch Photopolymerisation vernetzt werden, zeigen oft Phasenseparation. Ausserdem sind solche Materialien aufgrund ihres relativ hohen Gehaltes an Estergruppen oft nicht genügend stabil, um in gepufferter physiologischer Kochsalzlösung autoklaviert zu werden. Ferner zeigen solche Materialien (beispielsweise in Implantaten) unter physiologischen Bedingungen keine ausreichende Langzeitstabilität und können insbesondere enzymatisch leicht abgebaut werden.

Aus der FR-A-2641785 sind Polymermaterialien bekannt, die durch Umsetzung eines ungesättigten Polybutadiens, gegebenenfalls nach vorgängiger Aufpfropfung eines Monohydrodisiloxans, mit einem monomeren aromatischen Disilan erhältlich sind, sowie ihre Verwendung zur Herstellung von harten Kontaktlinsen. Die Sauerstoffdurchlässigkeit dieser vorbekannten Materialien wird mit 5,3 bzw. 12 Barrer-Einheiten angegeben.

Die zu lösende Aufgabe besteht nun darin, ein hoch sauerstoffdurchlässiges Material für die Herstellung von ophthalmischen Formkörpem anzubieten, das hervorragende mechanische und optische Eigenschaften aufweist, aber keine leicht spaltbaren Bindungen aufweist. Neben den bereits erwähnten Eigenschaften sollen solche Materialien auch gute Lagerstabilität, gute biologische Kompatibilität sowie gute Autoklavierbarkeit in gepufferter physiologischer Kochsalzlösung zeigen.

Das Problem wurde gelöst, indem ein ungesättigtes Polymer (a) mit einem Polysiloxan (b) vernetzt, und in einem zweiten optionalen Schritt mit einem vinylischen Monomer nach Bedarf pfropfcopolymerisiert wird.

Ein Gegenstand der vorliegenden Erfindung betrifft daher einen ophthalmischen Formkörper, welcher erhältlich ist, indem man ein ungesättigtes Polymer (a), das sich wiederholende Einheiten enthält, die ausgewählt sind aus Einheiten der Formel (I) und (II), 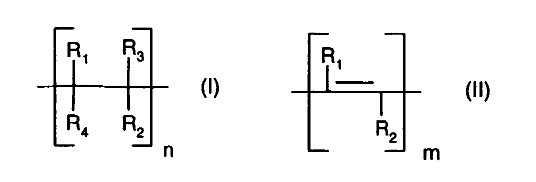 wobei
R₁ für Wasserstoff, Alkyl oder Trialkyl-silyl steht;
R₂ Alkyl, das unsubstituiert oder durch Alkoxy, Alkoxycarbonyl, Hydroxy, Hydroxycarbonyl, Halogen oder Aryl substituiert ist; Alkenyl, das unsubstituiert oder durch Alkoxy, Alkoxycarbonyl, Hydroxycarbonyl, Halogen oder Aryl substituiert ist; oder Alkinyl, das unsubstituiert oder durch Alkoxy, Alkoxycarbonyl, Hydroxycarbonyl, Halogen oder Aryl substituiert ist, bedeutet;
R₃ und R₄ unabhängig voneinander für Wasserstoff oder Alkyl stehen;
oder R₂ und R₃ zusammen für -(CH₂)_{q}- stehen, wobei q eine ganze Zahl von 3 bis 5 bedeutet, oder R₂ und R₃ zusammen für einen zweiwertigen Rest der Fomel (III) stehen, 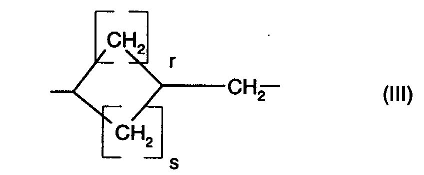 wobei r und s unabhängig voneinander für eine ganze Zahl von 1 bis 3 stehen; oder R₃ und R₄ zusammen für -(CH₂)ₚ- stehen, wobei p eine ganze Zahl von 3 bis 5 bedeutet; n und m unabhängig voneinander für eine ganze Zahl von 10 bis 100 000 stehen; und die Summe von n und m ebenfalls für eine ganze Zahl von 10 bis 100 000 steht, mit der Massgabe, dass mindestens 20 % aller Segmente mindestens je eine ungesättigte Kohlenstoff-Kohlenstoff-Bindung aufweisen;
und ein Polysiloxan (b) enthaltend Hydrosilangruppen der Formel (IV) 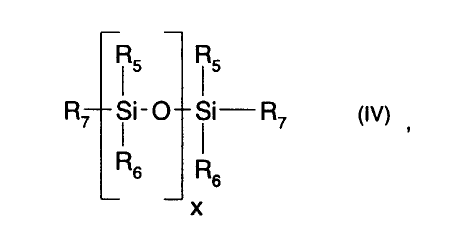 wobei der Index x für 1 bis 1000 steht; und
die Reste R₅, R₆ und R₇ unabhängig voneinander für Wasserstoff, Alkyl, Alkoxy, Phenyl oder fluorhaltiges Niederalkyl stehen, mit der Massgabe, dass in einer Verbindung der Formel (IV) mindestens zwei der Reste R₅, R₆ und R₇ und höchstens 20 % aller Reste R₅, R₆ und R₇ Wasserstoff bedeuten;
in solch einem Massenverhältnis umsetzt, dass das Vernetzungsprodukt daraus einen Polysiloxangehalt von 30 bis 80 Gew.-% aufweist.

Die Erfindung betrifft ferner einen ophthalmischen Formkörper aus einem Pfropfpolymer, welches durch Umsetzen eines oben beschriebenen Vernetzungsprodukts mit einem hydrophilen oder hydrophoben vinylischen Monomer erhältlich ist.

Ein ungesättigtes Polymer (a), das sich wiederholende Einheiten der Formel (I) und/oder (II) enthält, wird typischerweise durch einen Rest R₁, R₂, R₃ oder R₄ terminiert.

R₁ bedeutet bevorzugt Wasserstoff oder Niederalkyl mit bis zu 8 C-Atomen, bevorzugter Wasserstoff oder Niederalkyl mit bis zu 4 C-Atomen, stärker bevorzugt Wasserstoff oder Niederalkyl mit bis zu 2 C-Atomen und insbesondere Wasserstoff oder Methyl. Eine weitere bevorzugte Bedeutung von R₁ ist Triniederalkylsilyl und speziell Trimethylsilyl, insbesondere wenn R₁ an eine Einheit der Formel (II) geknüpft ist.

R₂ steht bevorzugt für Niederalkenyl mit bis zu 8 C-Atomen, das unsubstituiert oder durch Niederalkoxy, Niederalkoxycarbonyl, Hydroxycarbonyl, Halogen oder Phenyl substituiert ist; stärker bevorzugt Niederalkenyl mit bis zu 4 C-Atomen, das unsubstituiert oder durch Niederalkoxy, Niederalkoxycarbonyl, Hydroxycarbonyl, Halogen oder Phenyl substituiert ist; und insbesondere für Niederalkenyl mit bis zu 4 C-Atomen, das unsubstituiert oder durch Halogen oder Phenyl substituiert ist.

R₂ steht bevorzugt für Niederalkyl mit bis zu 8 C-Atomen, das unsubstituiert oder durch Niederalkoxy, Hydroxy, Halogen oder Phenyl substituiert ist; stärker bevorzugt Niederalkyl mit bis zu 4 C-Atomen, das unsubstituiert oder durch Niederalkoxy, Halogen oder Phenyl substituiert ist; und insbesondere für Niederalkyl mit bis zu 4 C-Atomen, das unsubstituiert oder durch Halogen oder Phenyl substituiert ist.

R₃ steht bevorzugt für Wasserstoff oder Niederalkyl mit bis zu 8 C-Atomen, stärker bevorzugt für Wasserstoff oder Niederalkyl mit bis zu 4 C-Atomen, speziell bevorzugt für Wasserstoff oder Niederalkyl mit bis zu 2 C-Atomen und insbesondere für Wasserstoff oder Methyl.

R₄ hat unabhängig von R₃ dieselbe Bedeutung und Bevorzugung.

R₂ und R₃ stehen in einer bevorzugten Bedeutung zusammen für -(CH₂)_{q}-, wobei q eine ganze Zahl von 3 bis 4 bedeutet, R₂ und R₃ stehen insbesondere bevorzugt für Trimethylen.

R₂ und R₃ stehen ebenfalls bevorzugt zusammen für einen zweiwertigen Rest der Fomel (III) steht, 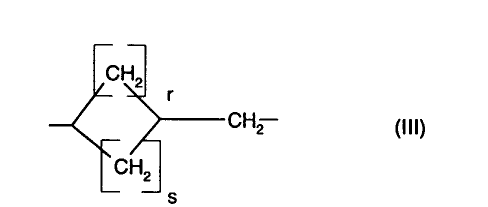 wobei r für eine ganze Zahl von 1 bis 3 und s für 2 steht.

R₃ und R₄ stehen in einer bevorzugten Bedeutung zusammen für -(CH₂)ₚ- wobei p eine ganze Zahl von 3 bis 4 bedeutet. R₃ und R₄ stehen insbesondere für Trimethylen.

Eine bevorzugte Bedeutung von n und m ist unabhängig voneinander eine ganze Zahl von 10 bis 100 000, stärker bevorzugt 20 bis 10 000 und insbesondere 25 bis 1000. Die Summe der Indices m und n steht ebenfalls bevorzugt für eine ganze Zahl von 10 bis 100 000, stärker bevorzugt für 20 bis 10 000 und insbesondere für 25 bis 1000.

Ein bevorzugtes ungesättigtes Polymer (a) ist eine Verbindung enthaltend sich wiederholende Einheiten, die ausgewählt sind aus Einheiten der Formel (I) und (II), worin R₁, R₃ und R₄ für Wasserstoff stehen und R₂ Niederalkenyl oder durch Halogen substituiertes Niederalkenyl bedeutet.

Ein bevorzugtes ungesättigtes Polymer (a) ist eine Verbindung enthaltend sich wiederholende Einheiten, die ausgewählt sind aus Einheiten der Formel (I) und (II), worin R₁, R₃ und R₄ für Wasserstoff stehen und R₂ für Niederalkenyl mit bis zu 4 C-Atomen steht.

Ein bevorzugtes ungesättigtes Polymer (a) ist eine Verbindung enthaltend sich wiederholende Einheiten der Formel (I), worin R₁, R₃ und R₄ für Wasserstoff stehen und R₂ Niederalkenyl mit bis zu 4 C-Atomen steht.

Ein bevorzugtes ungesättigtes Polymer (a) ist eine Verbindung enthaltend sich wiederholende Einheiten der Formel (II), worin R₁ für Triniederalkylsilyl und R₂ Niederalkyl steht.

Ein bevorzugtes ungesättigtes Polymer (a) ist eine Verbindung enthaltend alternierend sich wiederholende Einheiten der Formel (I) und (II), worin R₁, R₃ und R₄ für Wasserstoff stehen und R₂ für Niederalkyl oder Niederalkenyl mit bis zu 4 C-Atomen steht.

Ein ungesättigtes Polymer (a) ist beispielsweise ein Polymer eines konjugierten aliphatischen oder alicyclischen konjugierten Diens, das beispielsweise durch Halogen oder Niederalkyl substituiert ist; ein Polymer eines Alkins oder Dialkins, das unsubstituiert oder durch Niederalkyl oder Trimethylsilyl substituiert ist; ein Copolymer von einem konjugierten Dien mit einem hydrophilen oder hydrophoben vinylischen Monomer; ferner partiell hydrierte Derivate der genannten Verbindungen.

Beispiele für bevorzugte polymere konjugierte Diene sind cis-, trans-, iso- oder syndiotaktisches Poly-1,2-butadien, Poly-1,4-butadien oder Polyisopren; Poly-pentenamer; Polychloropren; Polypiperylen; Beispiele für Copolymere sind Butadien oder Isopren-Copolymere mit hydrophilen oder hydrophoben vinylischen Monomeren wie z.B. Acrylnitril, Styrol, Acrylsäure oder Hydroxyethylmethacrylat; ein Beispiele für ein Polyalkin ist Poly-1-trimethylsilyl-propin.

Ein stark bevorzugtes ungesättigtes Polymer (a) ist ausgewählt aus syndiotaktischem Poly-1,2-butadien, Poly-1,4-butadien und Polyisopren.

Ein stark bevorzugtes ungesättigtes Polymer (a) ist Poly-1-trimethylsilyl-propin.

Unter einem Polysiloxan (b) enthaltend Hydrosilangruppen wird im Rahmen der vorliegenden Erfindung bevorzugt ein Polysiloxan der zuvor angegebenen Formel (IV) verstanden, wobei der Index x für 1 bis 1000, insbesondere für 1 bis 500, vor allem für 1 bis 200 oder für 1 bis 100 und besonders bevorzugt für 2 bis 85 steht.

Im Zusammenhang mit den Resten R₅, R₆ und R₇ muss die Bedingung erfüllt sein, dass mindestens zwei von ihnen, aber nicht mehr als etwa 20 % aller Reste R₅, R₆ und R₇ Wasserstoff bedeuten. Besonders bevorzugt ist, dass nicht mehr als etwa 15 % aller Reste R₅, R₆ und R₇ Wasserstoff bedeuten. Speziell bevorzugt ist, dass genau zwei der Reste R₅, R₆ und R₇ Wasserstoff bedeuten. Dies sind bevorzugtermassen die beiden Reste R₇.

Eine bevorzugte Kombination von Bedeutungen, bei der zwei Silicium-Wasserstoff-Bindungen endständig angeordnet sind, ist die folgende:
Die Reste R₅ und R₆ bedeuten vorzugsweise Alkyl, fluorhaltiges Niederalkyl oder Phenyl, insbesondere Niederalkyl, wie Methyl oder Ethyl, oder Phenyl. Besonders bevorzugt ist für die Reste R₅ und R₆ die Bedeutung Niederalkyl mit bis zu 8 Kohlenstoffatomen, wie Methyl. Die Reste R₇ bedeuten Wasserstoff. Verbindungen der Formel (IV), bei denen die Reste R₅, R₆ und R₇ diese Bedeutungen aufweisen, sind α, ω-Dihydrogen-polysiloxane.

Eine andere bevorzugte Kombination von Bedeutungen, bei der Silicium-Wasserstoff-Bindungen nicht notwendigerweise endständig angeordnet sind, ist die folgende:

Die Reste R₅, R₆ und R₇ bedeuten Wasserstoff, fluorhaltiges Niederalkyl, Alkyl oder Phenyl, insbesondere Wasserstoff, Niederalkyl, wie Methyl oder Ethyl, oder Phenyl, mit der Massgabe, dass mindestens zwei der Reste R₅, R₆ und R₇ und vorzugsweise maximal 20 % der Reste R₅, R₆ und R₇ Wasserstoff bedeuten.

Ein ungesättigtes Polymer (a) und ein vorstehend genanntes Polysiloxan (b) enthaltend Hydrosilangruppen wird in An- oder Abwesenheit von einem Lösungsmittel und vorteilhafterweise in Anwesenheit eines Katalysators umgesetzt, was im Folgenden als Vernetzung bezeichnet wird.

Vorteilhafterweise wird ein Lösungsmittel verwendet, das sich weitgehend inert verhält, d.h. an der Reaktion nicht teilnimmt. Geeignete Beispiele hierfür sind Ether wie Tetrahydrofuran (THF), Dimethoxyethan, Diethylenglykoldimethylether oder Dioxan, halogenierte Kohlenwasserstoffe wie Chloroform oder Chlorbenzol, Kohlenwasserstoffe wie Toluol, Hexan, Methylcyclohexan oder Gemische mehrerer dieser Lösungsmittel. Die Menge an eingesetztem Lösungsmittel liegt bevorzugt in einem Bereich, dass Lösungen mit einer Konzentration von 1 bis 60 %, stärker bevorzugt 20 bis 60 % und insbesondere 5 bis 50 % Gewicht / Volumen resultieren, wobei sich die Gewichtsangabe auf die Menge an eingesetzten Reaktanden bezieht.

Als Katalysatoren für die Vemetzungsreaktion (oder Polyaddtionsreaktion) zwischen einem ungesättigten Polymer (a) und einem Polysiloxan (b) enthaltend Hydrosilangruppen, also einer Hydrosilylierungsreaktion, eignen sich vor allem Platin- und Rhodium-Komplexe oder Salze solcher Metalle wie z.B. H₂PtCl₆, PtCl₄ oder Platin-divinyltetramethyldisiloxan; ferner Peroxide wie z.B. Dibenzoylperoxid oder energiereiche Strahlung, wie z.B. UV-Licht geeigneter Wellenlänge. Die Menge an zugefügtem Katalysator liegt insbesondere bei flüssigen in einem Bereich von 0.0001 bis 1 Volumen % und vorteilhafterweise in einem Bereich von 0.001 bis 0.1 Volumen %. Bei festen Katalysatoren sind die genannten Zahlenbereiche Gewichts %.

Die Reaktanden, ein ungesättigtes Polymer (a) und ein Polysiloxan (b), werden in solch einem Massenverhältnis eingesetzt, dass ein Vemetzungsprodukt daraus ein Polysiloxangehalt von 30 - 80 Gew. % und insbesondere von 40 - 70 Gew. % aufweist.

Die Reaktionstemperatur der Vernetzung kann zum Beispiel von -30 bis 150°C betragen. Der Bereich von 10 bis 100°C ist ein bevorzugter Temperaturbereich. Ein weiterer bevorzugter Temperaturbereich ist die Raumtemperatur (RT). Die Reaktionszeiten liegen im Bereich von etwa 5 Minuten bis zu 48 Stunden, vorzugsweise etwa im Bereich von 0.5 bis 12 Stunden. Falls erforderlich wird unter Argon oder Stickstoff als Schutzgas gearbeitet

Die Reakfionmndpunktesfimmung erfolgt zweckmässigerweise durch eine Infrarot (IR) Absorbtionsmessung der Si-H Schwingung. Sobald die Si-H Bande im IR-Spektrum verschwindet, nähert sich die Vemetzung (Polyadditionsreaktion) dem Ende.

Ein vinylisches Monomer, das auf ein Vemetzungsprodukt, erhältlich durch Umsetzen eines ungesättigten Polymers (a) mit einem Polysiloxan (b) enthaltend Hydrosilangruppen, aufgepfropft wird, kann hydrophil oder hydrophob sein. Diese Reaktion wird Pfropfcopolymerisation genannt.

Eine Pfropfcopolymerisation wird typischerweise unter Zusatz eines Radikalbildners (oder Radikalinitiators) erwärmt. Ein solcher Radikalbildner ist z.B. Azadiisobutyronitril (AIBN), Kaliumperoxodisulfat, Dibenzoylperoxid, Wasserstoffperoxid oder Natriumpercarbonat. Werden beispielsweise die genannten Komponenten erwärmt, so bilden sich unter Homolyse Radikale, die dann typischerweise eine Pfropfcopolymerisation einleiten. Die Menge an zugesetztem Radikalbildner liegt typischerweise im Bereich von etwa 0.001 bis 5 Gew.% bezüglich der Gesamtmenge an Polymer, bevorzugt im Bereich von etwa 0.01 bis 2 Gew.% und insbesondere im Bereich von etwa 0.05 bis 1 Gew. %.

Eine Pkopfcopolymerisation kann auch mit energiereicher Strahlung, wie z.B. W-Licht geeigneter Wellenlänge ausgelöst werden, insbesondere unter Zusatz von geeigneten Photoinitiatoren wie z.B. Darocure®2959, Darocure®1171 oder Irgacure®184. Die Menge an zugesetztem Photoinitiator liegt typischerweise im Bereich von etwa 0.001 bis 10 Gew.% bezüglich der Gesamtmenge an Polymer, bevorzugt im Bereich von etwa 0.01 bis 5 Gew.% und insbesondere im Bereich von etwa 0.05 bis 2 Gew. %.

Eine Pfropfcopolymerisation kann in An- oder Abwesenheit von einem Lösungsmittel durchgeführt werden. Als Lösungsmittel sind grundsätzlich alle Lösungsmittel geeignet, die die verwendeten Monomere lösen, z.B. dipolar aprotische Lösungsmittel wie Dimethylsulfoxid, Acetonitril, N-Methylpyrrolidon (NMP) oder Dimethylacetamid, Ketone wie z.B. Methylethylketon oder Cyclohexanon, Ether wie z.B. THF, Dimethoxyethan oder Dioxan, Kohlenwasserstoffe wie z.B. Toluol oder Cyclohexan, ebenso Gemische von geeigneten Lösungsmitteln, wie z.B. Gemische von THF und Methylethylketon oder NMP und Cyclohexanon. Lösungen enthaltend ein vinylisches Monomer zum Aufpfropfen werden üblicherweise auf einen Bereich von 10 bis 70 % und bevorzugt auf einen Bereich von 20 bis 60 % Gewicht / Volumen eingestellt, wobei sich die Gewichtsangabe auf die Menge an eingesetztem Monomer bezieht. Wenn kein Lösungsmittel anwesend ist, kann beispielsweise ein vinylisches Monomer als Lösungsmittel per se fungieren, das idealerweise auch für die Propfcopolymerisation verwendet wird.

Ein Vemetzungsprodukt, auf welches ein vinylisches Monomer aufgepfropft werden soll, wird beispielsweise in einer Lösung, enthaltend ein vinylisches Monomer, beispielsweise für 2 bis 15 Minuten gequollen, aus der Lösung entfernt, und dann thermisch oder mit energiereicher Strahlung gemäss der nachstehenden Methoden pfropfcopolymerisiert.

Die Pfropfpolymerisation, in der therrnischen Variante, wird vorzugsweise in einem Temperaturbereich von 0 bis 150°C und stärker bevorzugt in einem Bereich von 20 bis 100°C durchgeführt. Die Reaktionszeiten liegen typischerweise im Bereich von etwa 5 Minuten bis zu etwa 12 Stunden, vorzugsweise in einem Bereich von 0.5 bis 4 Stunden. Falls erforderlich wird unter Argon oder Stickstoff als Schutzgas gearbeitet.

Wird die Pfropfpolymerisation unter dem Einfluss energiereicher Strahlung ausgeführt, so wird typischerweise mit UV-Licht geeigneter Wellenlänge bestrahlt, üblicherweise bei Raumtemperatur und typischerweise für etwa 2 bis 10 Minuten (z.B. mit einer 2000 Watt UV Hochdrucklampe). Falls erforderlich wird unter Argon oder Stickstoff als Schutzgas gearbeitet.

Das Ausmass, in dem ein vinylisches Monomer aufgepfropft wird, ist typischerweise abhängig von der Beschaffenheit eines Vernetzungsproduktes einerseits, und von dem vinylischen Monomer andererseits. Je mehr ungesättigte Kohlenstoff-Kohlenstoff Bindungen ein Vernetzungsprodukt im allgemeinen noch aufweist, desto mehr vinylisches Monomer kann üblicherweise auch aufgepfropft werden. Ein weiterer Faktor ist z.B. die sterische Hinderung sowohl beim Grundkörper wie auch bei einem vinylischen Monomer, die z.B. darüber entscheidet wieviel eines Monomers aufgepfropft werden kann.

Ein auf ein Vemetzungsprodukt (Primärpolymer) aufgepfropftes vinylisches Monomer stellt ein Sekundärpolymer dar, welches das Vemetzungsprodukt entweder teilweise oder vollständig durchdringt, oder es bildet eine aufgepfropfte Schicht. Die Schichtdicke eines Pfropfpolymers, die typischerweise durch die Dauer einer Pfropfcopolymerisation gesteuert werden kann, liegt im allgemeinen im Bereich von 1 bis 200 µm, bevorzugt im Bereich von 1 bis 150 µm und insbesondere im Bereich von 1 bis 100 µm.

Das Mengenverhältnis Primärpolymer zu Sekundärpolymer kann in weiten Grenzen variieren, es liegt bevorzugt in einem Bereich von 10 : 90 bis 90 : 10 Gew.%.

Wird ein Sekundärpolymer mit einem hydrophilen vinylischen Monomer aufgepfropft, so richtet sich die Menge von hydrophilen vinylischen Monomer bevorzugt nach dem Wassergehalt des Endproduktes, des Pfropfcopolymers, der z.B. bevorzugt bis zu 50 Gew.% und insbesondere von 15 - 35 Gew.% betragen soll.

Unter einem hydrophoben vinylischen Monomer wird ein Monomer verstanden, das als Homopolymer typischerweise ein Polymer ergibt, das wasserunlöslich ist und weniger als 10 Gewichtsprozent Wasser absorbieren kann.

Analog wird unter einem hydrophilen vinylischen Monomer ein Monomer verstanden, das als Homopolymer typischerweise ein Polymer ergibt, das wasserlöslich ist oder mindestens 10 Gewichtsprozent Wasser absorbieren kann.

Geeignete hydrophobe vinylische Monomere umfassen, ohne dass diese Auhählung abschliessend wäre, C₁-C₁₈-Alkyl- und C₃-C₁₈-cycloalkylacrylate und -methacrylate, C₃-C₁₈-Alkylacrylamide und -methacrylamide, Acrylnitril, Methacrylnitril, Vinyl-C₁-C₁₈-alkanoate, C₂-C₁₈-Alkene, C₂-C₁₈-Haloalkene, Styrol, Niederalkylstyrol, Niederalkylvinylether, C₂-C₁₀-Perfluoralkyl-acrylate und -methacrylate oder entsprechend partiell fluorierte Acrylate und Methacrylate, C₃-C₁₂-Perfluoralkyl-ethyl-thiocarbonylaminoethyl-acrylate und methacrylate, Acryloxy und Methacryloxy-alkylsiloxane, N-Vinylcarbazol, C₁-C₁₂-Alkylester der Maleinsäure, Fumarsäure, Itaconsäure, Mesaconsäure und dergleichen. Bevorzugt sind z.B. Acrylnitril, C₁-C₄-Alkylester von vinylisch ungesättigten Carbonsäuren mit 3 bis 5 Kohlenstoffatomen oder Vinylester von Carbonsäuren mit bis zu 5 Kohlenstoffatomen.

Beispiele geeigneter hydrophober vinylischer Monomere umfassen Methylacrylat, Ethylacrylat, Propylacrylat, Isopropylacrylat, Cyclohexylacrylat, 2-Ethylhexylacrylat, Methylmethacrylat, Ethylmethacrylat, Propylmethacrylat, Butylacrylat, Vinylacetat, Vinylpropionat, Vinylbutyrat, Vinylvalerat, Styrol, Chloropren, Isopren, Virtylchlorid, Vinylidenchlorid, Acrylnitril, 1-Buten, Butadien, Methacrylnitril, Vinyltoluol, Vinylethylether, Perfluorhexylethylthiocarbonylaminoethylmethacrylat, Isobornylmethacrylat, Trifluorethylmethacrylat, Hexafluorisopropylmethacrylat, Hexafluorbutylmethacrylat, Tris-trimethylsilyloxy-silylpropylmethacrylat, 3-Methacryloxypropylpentamethyldisiloxan und Bis(methacryloxypropyl)tetramethyldisiloxan.

Bevorzugte Beispiele hydrophober vinylischer Monomere sind, Tris-trimethylsilyloxysilylpropylmethacrylat, Methylmethacrylat, Trifluorethylmethacrylat und Hexafluorisopropylmethacrylat.

Geeignete hydrophile vinylische Monomere umfassen, ohne dass diese Aufzählung abschliessend wäre, durch Hydroxy substituierte Niederalkylacrylate und -methacrylate, Acrylamid, Methacrylamid, Niederalkylacrylamide und -methacrylamide, ethoxylierte Acrylate und Methacrylate, durch Hydroxy substituierte Niederalkylacrylamide und Methacrylamide, durch Hydroxy substituierte Niederalkylvinylether, Natriumvinylsulfonat, Natriumstyrolsulfonat, 2-Acrylamido-2-methylpropansulfonsäure, N-Vinylpyrrol, N-Vinyl-2-pyrrolidon, 2- und 4-Vinylpyridin, vinylisch ungesättigte Carbonsäuren mit insgesamt 3 bis 5 Kohlenstoffatomen, Aminoniederalkyl- (wobei der Begriff "Amino" auch quatemäres Ammonium umfasst), Mononiederalkylamino-niederalkyl- und Diniederalkylamino-niederalkylacrylate und -methacrylate, Allylalkohol und dergleichen. Bevorzugt sind z.B. N-Vinyl-2-pyrrolidon, Acrylamid, Methacrylamid, durch Hydroxy substituierte Niederalkyl(meth)acrylate, durch Hydroxy substituierte Niederalkylacrylamide und -methacrylamide und vinylisch ungesättigte Carbonsäuren mit insgesamt 3 bis 5 Kohlenstoffatomen.

Beispiele geeigneter hydrophiler vinylischer Monomere umfassen Hydroxyethylmethacrylat, Hydroxyethylacrylat, Hydroxypropylacrylat, Ammoniumethylmethacrylat-hydrochlorid, Acrylamid, Methacrylamid, N,N-Dimethylacrylamid, N-(meth)acryloylmorpholin, Allylalkohol, Vinylpyridin, Glycerinmethacrylat, N-(1,1-Dimethyl-3-oxobutyl)acrylamid, N-Vinyl-2-pyrrolidon, (Meth)acrylsäure, Methacrylsäure und dergleichen.

Bevorzugte hydrophile vinylische Monomere sind N,N-Dimethylacrylamid, N-Vinyl-2-pyrrolidon, Glycerinmethacrylat, (Meth)acrylsäure und N-(meth)acryloylmorpholin.

Unter einem vernetztem Polymer wird vor- und nachstehend sowohl ein Vemetzungsprodukt als auch ein Pfropfcopolymer verstanden, vorausgesetzt es wird nicht ausdrücklich etwas anderes gesagt. In einer bevorzugten Bedeutung wird unter einem vemetzten Polymer ein Pfropfcopolymer verstanden.

Alkyl weist bis zu 20 Kohlenstoffatome auf und kann geradkettig oder verzweigt sein. Alkyl steht bevorzugt für Niederalkyl, das bis zu 8 Kohlenstoffatome und weiter bevorzugt bis zu 4 und insbesondere bis zu 2 C-Atome aufweist. Geeignete Beispiele umfassen Dodecyl, Octyl, Hexyl, Pentyl, Butyl, Propyl, Ethyl, Methyl, 2-Propyl, 2-Butyl oder 3-Pentyl.

Aryl steht für eine carbocylische aromatische Verbindung mit bis zu 20 C-Atomen, die unsubstituiert oder durch vorzugsweise Niederalkyl oder Niederalkoxy substituiert ist. Beispiele sind Phenyl, Toluyl, Xylyl, Methoxyphenyl, t-Butoxyphenyl, Naphthyl oder Phenantryl.

Der Begriff "nieder bedeutet im Rahmen dieser Erfindung im Zusammenhang mit Resten und Verbindungen, soweit er nicht abweichend definiert ist, insbesondere Reste oder Verbindungen mit bis zu 8 Kohlenstoffatomen, vorzugsweise mit bis zu 6 und insbesondere mit bis zu 4 Kohlenstoffatomen.

Fluorhaltiges Niederalkyl steht für ein lineares oder verzweigtes Niederalkyl mit bis zu 8 Kohlenstoffatomen, das teilweise oder vollständig durch Fluor substituiert ist. Beispiele hiefür sind Trifluormethyl, Difluormethyl, Hexafluorpropyl oder 1,1 -Trifluorpropyl.

Alkenyl weist bis 2 bis 20 Kohlenstoffatome auf und kann linear oder verzweigt sein. Alkenyl steht insbesondere für Niederalkenyl mit 2 bis 8 C-Atomert, bevorzugt 2 bis 6 C-Atomert und insbesondere 2 bis 4 C-Atomen. Beispiele für Alkenyl sind Vinyl, Allyl, 1-Propen-2-yl, 1-Buten-2- oder -3- oder -4-yl, 2-Buten-3-yl, die Isomeren von Pentenyl, Hexenyl oder Octenyl.

Alkinyl weist bis 2 bis 20 Kohlenstoffatome auf und kann linear oder verzweigt sein. Alkinyl steht insbesondere für Niederalkinyl mit 2 bis 8 C-Atomert, bevorzugt 2 bis 6 C-Atomert und insbesondere 2 bis 4 C-Atomen. Beispiele für Alkinyl sind Ethinyl, Propargyl, 1-Butin-1-, 3-oder -4-yl, die Isomeren von Pentinyl, Hexinyl oder Octinyl.

Halogen steht insbesondere für Fluor, Chlor, Brom oder Jod, bevorzugt für Fluor, Chlor und Brom und speziell für Fluor und Chlor.

Alkoxy weist bis zu 20 Kohlenstoffatome auf und steht bevorzugt für Niederalkoxy. Niederalkoxy weist bis zu 8 Kohlenstoffatome auf, vorzugsweise bis zu 6 Kohlenstoffatome, und ist z.B. Methoxy, Ethoxy, Propoxy, Butoxy, tert.-Butoxy oder Hexyloxy.

Trialkylsilyl steht für Silyl das unabhängig voneinander drei Alkylliganden trägt. Trialkylsilyl steht bevorzugt für Triniederalkylsilyl. Beispiele für Triniederalkylsilyl sind t-Butyldimethylsilyl, Thexyl-dimethylsilyl oder Trimethylsilyl.

Die Erfindung betrifft ferner Verfahren zur Herstellung der erfindungsgemässen vemetzten Polymere. Ein ungesättigtes Polymer (a), beispielsweise Polybutadien, wird z.B. mit einem Polysiloxan-dihydrid, beispielsweise im Verhältnis 1 : 1 oder 2 :1, vermischt, wobei üblicherweise ein geeignetes Lösungsmittel verwendet wird; das resultierende Gemisch wird dann vorzugsweise entgast, üblicherweise mit einem geeigneten Katalysator versetzt und bevorzugt unter Schutzgas beispielsweise in eine Form, z.B. eine Kontaktlinsenform gefüllt; die Formen werden geschlossen und typischerweise während 0.5 - 12 Stunden auf 20 - 100°C erwärmt, bis alle H-Si Gruppen umgesetzt sind, was geeigneterweise mittels einer IR-spektoskopischen Messung bestimmt werden kann; schliesslich werden die vemetzten Formköper entfernt.

Diese Formkörper enthaltend ein (primäres, wie vorstehend beschriebenes) Vernetzungsprodukt können typischerweise in einem zweiten Schritt pfropfcopolymerisiert werden, z.B. wie folgt:
Die Formkörper werden z.B. in eine Lösung getaucht, die vinylisches Monomer sowie gegebenenfalls einen Pfropfcopolymerisationskatalysator enthalten, und für beispielsweise 10 Minuten gequollen; die gequollenen Formkörper werden im allgemeinen aus der Lösung entfernt, und schliesslich typischerweise unter Schutzgas erwärmt sowie, alternativ oder additiv dazu, mit z.B. UV-Licht geeigneter Wellenlänge bestrahlt.

Alternativ können die zwei Verfahrensschritte auch in umgekehrter Reihenfolge ausgeführt werden, nämlich z.B. folgendermassen:
Ein vinylisches Monomer kann zunächst auf ein ungesättigtes Polymer (a) aufgepfropft werden, typischerweise unter den vorstehend genannten Pfropfcopolymerisationsbedingungen, und kann dann in einem zweiten Schritt mit einem Polysiloxan (b), enthaltend Hydrosilangruppen, vernetzt werden, üblicherweise unter den vorstehend beschriebenen Vernetzungsbedingungen einer Hydrosilylierungsreaktion.

Die erfindungsgemässen Polymere können auf an sich bekannte Weise zu Formkörpern verarbeitet werden, insbesondere zu Kontaktlinsen, beispielsweise indem die Vernetzung der Polymere in einer geeigneten Kontaktlinsenform erfolgt. Weitere Beispiele für erfindungsgemässe Formkörper, neben Kontaktlinsen, sind z.B. künstliche Hornhaut, Intraokularlinsen, Augenverbände.

Kontaktlinsen, insbesondere weiche, die ein erfindungsgemässes vernetztes Polymer enthalten, weisen eine Palette ungewöhnlicher und äusserst vorteilhafter Eigenschaften auf. Unter diesen Eigenschaften sind beispielsweise ihre ausgezeichnete Verträglichkeit mit der menschlichen Cornea (gegebenenfalls nach einer geeigneten Oberflächenbehandlung (z.B. Plasma-Hydrophilierung)) sowie mit der Tränenflüssigkeit zu nennen, die auf einem ausgewogenen Verhältnis von Wassergehalt, Sauerstoffdurchlässigkeit und mechanischen sowie adsorptiven Eigenschaften beruhen. Aufgrund ihrer günstigen Permeabilitätseigenschaffen bezüglich verschiedenartiger Salze, Nährstoffe, Wasser sowie diverser anderer Komponenten der Tränenflüssigkeit und Gase (CO₂, O₂) beeinträchtigen erfindungsgemässe Kontaktlinsen die natürlichen metabolischen Prozesse in der Cornea nur unwesentlich oder gar nicht. Im Gegensatz zu vielen Siloxan-haltigen Kontaktlinsen aus dem Stand der Technik, zeigen Linsen, die ein erfindungsgemässes vernetztes Polymer als wesentlichen Bestandteil enthalten hohe Formbeständigkeit und ausgezeichnete Lagerstabilität.

Ein bevorzugte Ausführungsform betrifft Kontaktlinsen, die im wesentlichen oder vollständig aus einem erfindungsgemässen Pfropfcopolymer bestehen, wobei der Wassergehalt insbesondere in einem Bereich von 15 - 30 Gew.% liegt.

Erfindungsgemässe Kontaktlinsen mit einem bevorzugten Wassergehalt in einem Bereich von 15 - 30 Gew.% zeigen auch eine hohe Wasserdurchlässigkeit und ferner eine vorteilhafte Beweglichkeit auf dem Auge.

Eine weitere bevorzugte Ausführungsform betrifft Kontaktlinsen, die im wesentlichen aus einem erfindungsgemässen vernetzten Polymer bestehen, wobei es sich um schwach wasserhaltige, flexible gasdurchlässige (RGP) Kontaklinsen handelt.

Alle die vorstehend genannten Vorteile gelten naturgemäss nicht nur für Kontaktlinsen, sondern auch für andere erfindungsgemässe Formkörper.

Ein weiterer Gegenstand der vorliegenden Erfindung ist gerichtet auf die Verwendung eines erfindungsgemässen vemetzten Polymers für die Beschichtung von ophthalmisch verwendbaren Polymersubstraten, wie z.B. Produkten wie Kontaktlinsen, Intraokularlinsen oder Augenverbänden, wobei hydrophile Beschichtungen (Coatings) bevorzugt sind.

Ein erfindungsgemässes vemetztes Polymer ist auch geeignet, um als Hornhautimplantat oder künstliche Hornhaut (comeal implant, artificial cornea) verwendet zu werden; ferner als Zellwachstumssubstrate, als Material für die Befestigung und die Züchtung von Tierzellen in vitro und in vivo, als medizinische Implantate wie z.B. implantierbare semipermeable Membranmaterialien, als Gewebsimplantate für die kosmetische Chirurgie, als Implantat, das hormonausscheidende Zellen enthält, wie z.B. Pankreas-lnselzellen, als Brustimplantat oder als künstliches Gelenk und dergleichen.

Ein weiterer Gegenstand der vorliegenden Erfindung ist daher gerichtet auf ein Hornhautimplantat, das aus einem vorstehend beschriebenen vernetzten Polymer hergestellt ist. Ein genanntes Hornhautimplantat kann mit dem gleichen Verfahren hergestellt werden, wie vorstehend bei der Herstellung von Kontaktlinsen beschrieben worden ist. Hornhautimplantate können mit konventionellen chirurgischen Verfahren eingepflanzt werden, z.B. unter, in oder durch das Epithelgewebe der Hornhaut oder in die Stroma der Hornhaut oder in andere Gewebsschichten der Hornhaut. Solche Implantate können die optischen Eigenschaften der Hornhaut ändern, z.B. im Sinne einer Korrektur eines visuellen Defizits und / oder indem das Erscheinungsbild des Auges verändert wird, wie z.B. die Pupillenfärbung. Ein Hornhautimplantat kann das Gebiet über der optischen Achse umfassen, das bei der Implantation die Pupille bedeckt und Sehfähigkeit vermittelt, ferner das Gebiet, das die Peripherie der optischen Achse umgibt. Das Implantat kann über den gesamten Bereich die gleichen visuellen Eigenschaften besitzen.

Es wurde gefunden, dass der Durchfluss von hochmolekularen Komponenten von Gewebeflüssigkeit, beispielsweise von Proteinen oder Glykoproteinen, wie z.B. Wachstumsfaktoren, Peptide, Hormone oder Proteine, die verantwortlich sind für den Transport von essentiellen Metallionen durch ein Hornhautimplantat, insbesondere zwischen Epithelzellen und Stromazellen und selbst hinter die Endothelschicht, wichtig ist sowohl für ein Überleben von Gewebe als auch für die Lebensfähigkeit von Gewebe ausserhalb und innerhalb eines Hornhautimplantats. Ein Homhautimplantat wird daher bevorzugt hergestellt mit einer Porosität, die ausreicht, um Flüssigkeitskomponenten von Gewebe mit einem Molekulargewicht von > 100'000 Dalton durchzulassen, wobei ein Durchfluss von Komponenten von Gewebsflüssigkeit gewährleistet ist zusätzlich zu einem Durchfluss von niedermolekularen Nährstoffkomponenten wie z.B. Glucose, Fette oder Aminosäuren oder Atemgase zwischen Zellen beidseits eines Implantates.

Die Porosität eines Hornhautimplantates ist entweder gegeben durch das Polymermaterial selbst aus dem es hergestellt wird. Andererseits können Poren zusätzlich in ein erfindungsgemässes vernetztes Polymer eingebaut werden, nämlich durch eines der zahlreichen bekannten Verfahren, die beispielsweise beschrieben sind in WO 90/07575, WO91/07687, US5'244'799, US5'238'613, US 4'799'931 oder US 5'213'721.

Gleichgültig mit welcher Methode die erforderliche Porosität eines erfindungsgemässen Implantates ausgebildet wird, ein Implantat hat vorzugsweise eine Porosität die ausreicht, um Proteine und andere biologische Makromoleküle mit einem Molekulargewicht bis zu oder grösser als 10'000 Dalton durchzulassen, wie beispielsweise ein Molekulargewicht von 10'000 - 1'000'000 Dalton, aber nicht so gross, dass ganze Zellen passieren können und in das Gebiet über der optischen Achse des Implantates eindringen können. Wo die Porosität des Implantates durch Poren ermöglicht wird, enthält das Gebiet über der optischen Achse eine Vielzahl von Poren, dessen Zahl nicht beschränkt sein soll, die aber ausreichen soll, um zwischen dem äusseren und dem inneren Gebiet eines Implantates freien Durchfluss von Gewebskomponenten zu ermöglichen. Die Poren, die über dem Gebiet der optischen Achse liegen, verursachen vorzugsweise keine Streuung von sichtbarem Licht in einem Ausmass, das sich als problematisch hinsichtlich einer Sehkorrektur auswirken würde. Mit dem Begriff einer Pore wird vor- und nachstehend eine Pore verstanden, die keine geometrische Einschränkungen aufweist und sowohl eine regelmässige als auch eine unregelmässige Morphologie aufweist. Die Angabe einer Porengrösse bedeutet nicht, dass alle Poren den gleichen Durchmesser aufweisen. Vielmehr handelt es sich um einen durchschnittlichen Durchmesser.

Auf dem Gebiet ausserhalb der optischen Achse kann das Hornhautimplantat die gleiche Porosität aufweisen wie im Gebiet über der optischen Achse. Dieses periphere Gebiet eines Implantates, das das Gebiet der optischen Achse umgibt, wird auch als Schürze (skirt) bezeichnet, und kann aber im Gegensatz zum Gebiet der optischen Achse ein Einwachsen von Hornhautzellen zulassen, wodurch eine Verankerung des Implantates am Auge geschieht.

Die Porosität in der Schürze kann auch ein eigenständiges Merkmal des Materials sein, aus dem die Schürze hergestellt wird. Ist die Schürze aus dem gleichen Material wie das Material über der optischen Achse, so können Poren mit unterschiedlichem Durchmesser einerseits an der Schürze und zum andern über der optischen Achse eingebaut werden. Andererseits kann die Schürze aus einem andern Material hergestellt werden als das Material über der optischen Achse, wobei wie vorstehend ausgeführt wurde, die Porosität in der Schürze grösser sein soll als jene über der optischen Achse. Eine Schürze besteht vorzugsweise aus einem optisch klaren Polymer wie eines über der optischen Achse; die Schürze kann aber auch aus einem optisch nicht-klaren Material bestehen, oder sie wird hergestellt aus porösem Material, das optisch nicht klar ist.

Ein erfindungsgemässes vernetztes Polymer kann die Besiedelung mit Gewebezellen wie beispielsweise vaskuläre Endothelzellen, Fibroplasten oder in Knochen gebildete Zellen unterstützen, wobei es nicht notwendig ist, dass eine spezifische Oberflächenbeschaffenheit vorhanden ist, um die Zelladhäsion und das Zellwachstum zu stimulieren. Dies ist vorteilhaft, da die Verfahrenskosten klein gehalten werden können. Andererseits kann ein erfindungsgemässes vernetztes Polymer mit einer bekannten Technik an seiner Oberfläche modifiziert werden, wie z.B. die Plasmabehandlung einer Oberfläche mittels Radiofrequenz Glimmentladung, beispielsweise wie in US 4'919'659 oder in WO 89/00220 beschrieben, oder durch Bestrahlung oder mit einer chemischen Behandlung.

Ein erfindungsgemässes vernetztes Polymer kann mit einer oder mit mehreren Komponenten auf seiner Oberfläche beschichtet werden, um beispielsweise das Wachstum von Gewebe zu fördern. Solche Materialien sind beispielsweise Fibronectin, Chondroitansulfat, Collagen, Laminin, Zellanheftungsproteine, Globulin, Chondronectin, epidermale Wachstumsfaktoren, Muskelfaserproteine, und / oder Derivate davon, aktive Fragmente und Mischungen davon. Fibronectin, epidermale Wachstumsfaktoren und / oder deren Derivate, aktive Fragmente und Mischungen davon sind speziell nützlich. Eine solche Oberflächenbeschichtung kann falls erforderlich auch nach einer oben beschriebenen Oberflächenmodifikation vorgenommen werden. Ein erfindungsgemässes vernetztes Polymer kann vorteilhafterweise mehrere der genannten Eigenschaften in sich vereinigen, wie z.B. das Anheften an Zellen mit guter Biostabilität sowie Resistenz gegenüber Ablagerungen.

Die mechanischen Eigenschaften eines erfindungsgemässen vernetzten Polymers sind geeignet, um als Hornhautimplantat verwendet zu werden, wobei das Material vorzugsweise einen Elastizitätsmodul von 0.5 - 10 MPa aufweist. Ein genannter Elastizitätsmodul verleiht einem Hornhautimplantat eine geeignete Flexibilität, um das Einfügen ins Auge zu ermöglichen, wie beispielsweise über dem Gebiet der Bowman'schen Membran.

Ein erfindungsgemässes vernetztes Polymer kann ferner verwendet werden als Zellwachstumsunterlage, z.B. als Zellkulturgerät, z.B. Geschirr, Flaschen, Schalen und ähnliches, ferner in biologischen Reaktoren wie beispielsweise in der Herstellung von wertvollen Proteinen und anderen Zellkulturkomponenten.

Die nachfolgenden Beispiele erläutern den Gegenstand der Erfindung, ohne ihn jedoch etwa auf den Umfang der Beispiele zu beschränken. Prozente bei Mengenangaben sind Gewichtsprozente soweit nicht ausdrücklich anders angegeben. Temperaturen sind in Grad Celsius angegeben.

### Beispiel 1

2.2 g Poly(1,2-syndiotaktisches)-butadien (Polysciences Inc.) mit einem mittleren Molekulargewicht von 100'000 g/Mol werden in einem Dreihalskolben vorgelegt. Unter Stickstoff und unter Rühren werden 50 ml absolutes Dimethoxyethan (DME) zugegeben und kurz auf 60°C erwärmt. Dann wird auf Raumtemperatur (RT) gekühlt und eine Lösung von 1.1 g H - Siloxan (Versuchsprodukt 1085 von Goldschmidt AG) in 5 ml absolutem DME langsam zugegeben. Es wird auf 40°C erwärmt, so dass eine homogene Lösung entsteht (ca. 10 Minuten). Es wird erneut auf RT gekült und dann 1 Stunde lang Stickstoff durch diese Mischung geleitet. Danach wird auf 40°C erwärmt und unter Rühren 2 Tropfen des Katalysators Platin-divinyltetramethyl-disiloxan (PC 072, ABCR) zugegeben. Nach 5 Minuten Rühren wird mit dieses Reaktionsgemisch zu einem 1.0 mm dicken Film zwischen zwei Glasplatten gegossen. Dieser Film wird zwischen den Glasplatten unter Stickstoff bei 60°C für 16 Stunden belassen. Dann wird auf RT gekühlt, die Glasplatten werden entfernt, der verbleibende hochelastische, optisch klare Film wird mit Ethanol extrahiert und dann bis zur Gewichtskonstanz getrocknet.

### Beispiel 2

In Analogie zu Beispiel 1 wurden 2.2 g Poly(1,2-syndiotaktisches)-butadien und 4.4 g H - Siloxan (Versuchsprodukt K-3272, Goldschmidt AG) in DME umgesetzt.
Der Film dieses Beispiels ist ziemlich spröde.

### Beispiel 3

1.1 g Poly(1,2-syndiotaktisches)-butadien werden in 20 ml absolutem THF und unter Erwärmen auf 40°C unter Stickstoff gelöst. Hierzu werden 1,1 g H - Siloxart (Versuchsprodukt 1085 von Goldschmidt AG) gegeben und bei dieser Temperatur 10 Minuten ausgerührt. Dann wird auf RT gekühlt und entgast, indem man noch eine Stunde lang Stickstoff durch die Lösung leitet. Hierauf werden 2 Tropfen des Katalysators Platin-divinyltetramethyl-disiloxan (PC 072, ABCR) zugegeben und 5 Minuten verrührt. Danach wird ein 1.0 mm dicker Film zwischen 2 Glasplatten gegossen. Der Film wird zwischen den Glasplatten unter Stickstoff bei 60°C für 16 Stunden belassen. Dann wird auf RT gekühlt, die Glasplatten werden entfernt, der verbleibende Film wird einmal mit THF und zweimal mit Isopropanol extrahiert und dann bis zur Gewichtskonstanz getrocknet.

### Beispiel 4

2.2 g Poly(1,2-syndiotaktisches)-butadien werden in 30 ml Methylcyclohexan (MCH) bei 40°C unter Stickstoff gelöst. Eine Lösung von 2 g H - Siloxan (Versuchsprodukt K-3272, Goldschmidt AG) in 5 ml MCH wird langsam zugegeben und für 5 Minuten verrührt. Dann wird auf RT gekühlt und für 30 Minuten Stickstoff durch die Lösung geleitet. Danach gibt man 3 Tropfen des Katalysators Platin-divinyltetramethyl-disiloxan (PC 072, ABCR) in 1 ml MCH zu., und erwärmt auf 50°C unter Rühren für 3 Minuten. Danach wird ein 1.5 mm dicker Film zwischen 2 Glasplatten gegossen. Der Film wird zwischen den Glasplatten unter Stickstoff bei 60°C für 16 Stunden belassen. Dann wird auf RT gekühlt, die Glasplatten werden entfernt, der verbleibende Film wird mit THF extrahiert und dann bis zur Gewichtskonstanz getrocknet.

### Beispiel 5

In Analogie zu Beispiel 4 wird ein Polymerfilm hergestellt mit den folgenden Komponenten: 3.3 g Poly(1,2-syndiotaktisches)-butadien und 2.0 g H - Siloxan (Versuchsprodukt K-3272, von Goldschmidt AG).

### Beispiel 6

In Analogie zu Beispiel 4 wird ein Polymerfilm hergestellt mit den folgenden Komponenten: 3.0 g Poly(1,2-syndiotaktisches)-butadien und 2.0 g H - Siloxan (Versuchsprodukt 1085 von Goldschmidt AG).

### Beispiel 7

7.0 g Hydroxylterminiertes Polybutadien (Polysciences Inc., Katalog Nr. 06508, mittleres Molekulargewicht ca. 2800) werden unter Stickstoff und Rühren in 9 ml MCH gelöst. In diese Lösung werden 3.5 g H - Siloxan (Versuchsprodukt 1085 von Goldschmidt AG) eingerührt. Dann wird für 30 Minuten Stickstoff durch die Lösung geleitet. Hierzu gibt man 3 Tropfen des Katalysators Platin-divinyltetramethyl-disiloxan (PC 072, ABCR) und rührt weitere 10 Minuten. Mit einem Teil dieser Lösung werden Polypropylen Kontaktlinsen-Förmchen (Molds) mit einem Durchmesser von 21 mm und einer Mittenhöhe von 0.5 mm gefüllt und dann mit einem lose sitzenden Polypropylendeckel verschlossen. Diese Förmchen werden für 12 Stunden unter Stickstoff erwärmt (60°C).
Danach werden die Förmchen geöffnet, die Linsen entfernt, mit THF/Aceton (1:1) extrahiert und bis zur Gewichtskonstanz getrocknet.

### Beispiel 8

In Analogie zu Beispiel 7 werden Linsen hergestellt, ausgehend von 6 g Hydroxyl-terminiertem Polybutadien (Polysciences Inc., Katalog Nr. 06508) und 4.0 g H-Siloxan (Versuchsprodukt 1085 von Goldschmidt AG).

| Eigenschaften der Polymere aus den Beispielen | | | | |
|---|---|---|---|---|
| Beispiel | Polybutadien (PB) mittleres MG | H-Siloxan (HS) mittleres MG | Verhältnis PB / HS | Shore A Härte |
| 1 | 100'000 | 566 | 2:1 | 42 |
| 3 | 100'000 | 566 | 1:1 | 43 |
| 4 | 100'000 | 2200 | 1:1 | 52 |
| 5 | 100'000 | 2200 | 3:2 | 62 |
| 6 | 100'000 | 566 | 3:2 | 46 |
| 7 | 3000 | 566 | 2:1 | 63 |
| 8 | 3000 | 566 | 3:2 | 57 |

| Eigenschaften der Polymere aus den Beispielen | | | | |
|---|---|---|---|---|
| Beispiel | E-Modulus (MPa) | O₂Dk (Barrer) | Bruchdehnung (%) | Extrahierbare Anteile (%) |
| 1 | 1.27 | 152 | 370 | 3.1 |
| 3 | 0.74 | 105 | 80 | 3.5 |
| 4 | 1.13 | 196 | 420 | 2.5 |
| 5 | 1.21 | 159 | 430 | 2.7 |
| 6 | 0.82 | 129 | 174 | 4.5 |
| 7 | 1.24 | 57 | 52 | 2.9 |
| 8 | 0.84 | 55 | 67 | 4.71 |

### Beispiel 9

In Analogie zu Beispiel 4 wird ein Polymerfilm hergestellt und ein 2 x 1 cm grosses Stück davon für 10 Minuten in eine Lösung getaucht, die 2.6 g N-Vinylpyrrolidon (NVP), 6.4 g Methylethylketon und 0.2 g Irgacure®184 (Photoinitiator Ciba-Geigy) enthält. Der gequollene Film wird dann entnommen und mit Methylethylketon abgespült und mit Fliesspapier abgetrocknet. Der so präparierte Film wird beidseitig je 2 Minuten mit UV (2000 W) bestrahlt. Dann wird der Film für 12 Stunden mit Methylethylketon extrahiert und danach bis zur Gewichtskonstanz getrocknet.

| Kontaktwinkel | Advancing Angle | Receding Angle |
|---|---|---|
| vor der NVP-Modifikation | 111 | 71 |
| nach der NVP-Modifikation | 97 | 38 |

### Beispiel 10

In Analogie zu Beipiel 9 wurde der Polymerfilm aus Beispiel 4 mit einer Lösung behandelt, die 2.6 g Dimethylacrylamid (DMA).4 g Methylethylketon und 0.2 g Irgacure®184 (Photoinitiator Ciba-Geigy) enthält. Es wird wie in Beispiel 9 zehn Minuten getaucht, gespült und mit UV bestrahlt.

| Kontaktwinkel | Advancing Angle | Receding Angle |
|---|---|---|
| vor der DMA-Modifikation | 111 | 71 |
| nach der DMA-Modifikation | 92 | 44 |

## Patentansprüche

1. Ophthalmischer Formkörper, erhältlich durch Umsetzen eines ungesättigten Polymers (a), das sich wiederholende Einheiten enthält, die ausgewählt sind aus Einheiten der Formel (I) und (II), 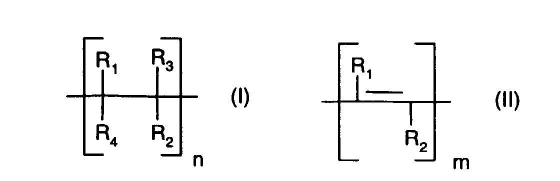 wobei
R₁ für Wasserstoff, Alkyl oder Trialkyl-silyl steht;
R₂ Alkyl, das unsubstituiert oder durch Alkoxy, Alkoxycarbonyl, Hydroxy, Hydroxycarbonyl, Halogen oder Aryl substituiert ist; Alkenyl, das unsubstituiert oder durch Alkoxy, Alkoxycarbonyl, Hydroxycarbonyl, Halogen oder Aryl substituiert ist; oder Alkinyl, das unsubstituiert oder durch Alkoxy, Alkoxycarbonyl, Hydroxycarbonyl, Halogen oder Aryl substituiert ist, bedeutet;
R₃ und R₄ unabhängig voneinander für Wasserstoff oder Alkyl stehen;
oder R₂ und R₃ zusammen für -(CH₂)_{q}- stehen, wobei q eine ganze Zahl von 3 bis 5 bedeutet, oder R₂ und R₃ zusammen für einen zweiwertigen Rest der Fomel (III) stehen, 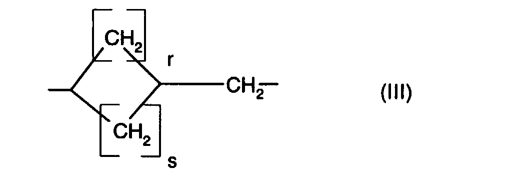 wobei r und s unabhängig voneinander für eine ganze Zahl von 1 bis 3 stehen; oder
R₃ und R₄ zusammen für -(CH₂)ₚ- stehen, wobei p eine ganze Zahl von 3 bis 5 bedeutet; n und m unabhängig voneinander für eine ganze Zahl von 10 bis 100 000 stehen; und die Summe von n und m ebenfalls für eine ganze Zahl von 10 bis 100 000 steht, mit der Massgabe, dass mindestens 20 % aller Segmente mindestens je eine ungesättigte Kohlenstoff-Kohlenstoff-Bindung aufweisen;
und eines Polysiloxans (b) enthaltend Hydrosilangruppen der Formel (IV) 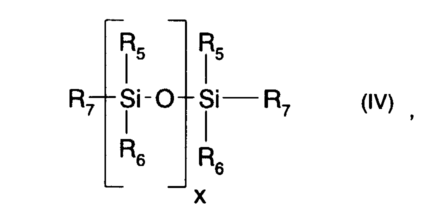 wobei der Index x für 1 bis 1000 steht; und die Reste R₅, R₆ und R₇ unabhängig
voneinander für Wasserstoff, Alkyl, Alkoxy, Phenyl oder fluorhaltiges Niederalkyl mit bis zu 8 Kohlenstoffatomen stehen, mit der Massgabe, dass in einer Verbindung der Formel (IV) mindestens zwei der Reste R₅, R₆ und R7 und höchstens 20 % aller Reste R₅, R₆ und R₇ Wasserstoff bedeuten; in solch einem Massenverhältnis, dass das Vernetzungsprodukt daraus einen Polysiloxangehalt von 30 bis 80 Gew.-% aufweist.

2. Ophthalmischer Formkörper aus einem Pfropfpolymer, das durch Umsetzen eines Vernetzungsprodukts gemäss Anspruch 1 mit einem hydrophilen oder hydrophoben vinylischen Monomer erhältlich ist.

3. Ophthalmischer Formkörper gemäss Anspruch 1 oder 2, wobei das ungesättigte Polymer (a) sich wiederholende Einheiten enthält, die ausgewählt sind aus Einheiten der Formel (I) und (II), worin R₁, R₃ und R₄ für Wasserstoff stehen und R₂ Niederalkenyl oder durch Halogen substituiertes Niederalkenyl mit bis zu 8 C-Atomen bedeutet.

4. Ophthalmischer Formkörper gemäss Anspruch 1 oder 2, wobei das ungesättigte Polymer (a) sich wiederholende Einheiten enthält, die ausgewählt sind aus Einheiten der Formel (I) und (II), worin R₁, R₃ und R₄ für Wasserstoff stehen und R₂ für Niederalkenyl mit bis zu 4 C-Atomen steht.

5. Ophthalmischer Formkörper gemäss Anspruch 1 oder 2, wobei das ungesättigte Polymer (a) sich wiederholende Einheiten der Formel (I) enthält, worin R₁, R₃ und R₄ für Wasserstoff stehen und R₂ für Niederalkenyl mit bis zu 4 C-Atomen steht.

6. Ophthalmischer Formkörper gemäss Anspruch 1 oder 2, wobei das ungesättigte Polymer (a) sich wiederholende Einheiten der Formel (II) enthält, worin R₁ für Triniederalkylsilyl und R₂ für Niederalkyl steht.

7. Ophthalmischer Formkörper gemäss Anspruch 1 oder 2, wobei das ungesättigte Polymer (a) alternierend sich wiederholende Einheiten der Formel (I) und (II) enthält, worin R₁, R₃ und R₄ für Wasserstoff stehen und R₂ für Niederalkyl oder Niederalkenyl mit bis zu 4 C-Atomen steht.

8. Ophthalmischer Formkörper gemäss Anspruch 1 oder 2, wobei das ungesättigte Polymer (a) ausgewählt ist aus syndiotaktischem Poly-1,2-butadien, Poly-1,4-butadien und Polyisopren.

9. Ophthalmischer Formkörper gemäss einem der Ansprüche 1 bis 8, wobei das Polysiloxan (b) für eine Verbindung der Formel (IV) steht, worin genau zwei der Reste R₅, R₆ und R₇ Wasserstoff bedeuten.

10. Ophthalmischer Formkörper gemäss Anspruch 9, wobei die beiden Reste R₇ Wasserstoff bedeuten.

11. Ophthalmischer Formkörper gemäss einem der Ansprüche 1 bis 10, wobei das Polysiloxan (b) für eine Verbindung der Formel (IV) steht, zwei Silicium-WasserstoffBindungen endständig angeordnet sind und die Reste R₅ und R₆ für Niederalkyl, fluorhaltiges Niederalkyl oder Phenyl stehen.

12. Ophthalmischer Formkörper gemäss Anspruch 11, wobei die Reste R₅ und R₆ für Niederalkyl mit bis zu 8 Kohlenstoffatomen und insbesondere für Methyl stehen.

13. Ophthalmischer Formkörper gemäss einem der Ansprüche 1 bis 12, wobei x für 1 bis 500, vor allem für 1 bis 200 oder für 1 bis 100 und besonders bevorzugt für 2 bis 85 steht.

14. Ophthalmischer Formkörper gemäss Anspruch 1 oder 2, wobei das ungesättigte Polymer (a) ausgewählt ist aus der Gruppe eines polymeren konjugierten aliphatischen oder alicyclischen konjugierten Diens, das durch Halogen oder Niederalkyl substituiert ist; eines Polymers eines Alkins oder Dialkins, das unsubstituiert oder durch Niederalkyl oder Trimethylsilyl substituiert ist; eines Copolymers von einem konjugierten Dien mit einem hydrophilen oder hydrophoben vinylischen Monomer; oder von einem partiell hydrierten Derivat der genannten Verbindungen.

15. Ophthalmischer Formkörper gemäss einem der Ansprüche 2 bis 14, wobei das hydrophobe vinylische Monomer ausgewählt ist unter C₁-C₁₈-Alkyl- und C₃-C₁₈-Cycloalkylacrylat und -methacrylat, C₃-C₁₈-Alkylacrylamid und -methacrylamid, Acrylnitril, Methacrylnitril, Vinyl-C₁-C₁₈-alkanoat, C₂-C₁₈-Alken, C₂-C₁₈-Haloalken, Styrol, Niederalkylstyrol, Niederalkylvinylether, C₂-C₁₀-Perfluoralkyl-acrylat und methacrylat oder entsprechend partiell fluoriertes Acrylat und Methacrylat, C₃-C₁₂-Perfluoralkyl-ethyl-thiocarbonylaminoethyl-acrylat und methacrylat, Acryloxy und Methacryloxy-alkylsiloxan, N-Vinylcarbazol und C₁-C₁₂-Alkylester der Maleinsäure, Fumarsäure, Itaconsäure und Mesaconsäure.

16. Ophthalmischer Formkörper gemäss einem der Ansprüche 2 bis 14, wobei das hydrophile vinylische Monomer ausgewählt ist unter durch Hydroxy substituiertes Niederalkylacrylat und -methacrylat, Acrylamid, Methacrylamid, Niederalkylacrylamid und - methacrylamid, ethoxyliertes Acrylat und Methacrylat, durch Hydroxy substituiertes Niederalkylacrylamid und Methacrylamid, durch Hydroxy substituierter Niederalkylvinylether, Natriumvinylsulfonat, Natriumstyrolsulfonat, 2-Acrylamido-2-methylpropansulfonsäure, N-Vinylpyrrol, N-Vinyl-2-pyrrolidon, 2- und 4-Vinylpyridin, vinylisch ungesättigte Carbonsäuren mit insgesamt 3 bis 5 Kohlenstoffatomen, Aminoniederalkyl-, Mononiederalkylamino-niederalkyl- und Diniederalkylamino-niederalkylacrylat und -methacrylat und Allylalkohol.

17. Ophthalmischer Formkörper gemäss einem der Ansprüche 1 bis 16, dadurch gekennzeichnet, dass es sich um eine Kontaktlinse, ein Hornhautimplantat oder um eine künstliche Hornhaut handelt.

18. Verwendung eines Polymers gemäss einem der Ansprüche 1 bis 16 zur Herstellung von Kontaktlinsen.

19. Verwendung eines Polymers gemäss einem der Ansprüche 1 bis 16 zur Beschichtung von ophthalmisch verwendbaren Polymersubstraten.

20. Verwendung eines Polymers gemäss einem der Ansprüche 1 bis 16 als Hornhautimplantat oder künstliche Homhaut.

## Claims

1. An ophthalmic moulding obtainable by reacting an unsaturated polymer (a) that contains repeating units selected from units of formulae (I) and (II) 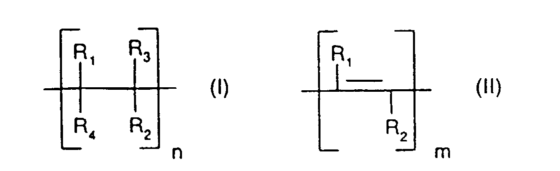 wherein
R₁ is hydrogen, alkyl or trialkylsilyl;
R₂ is alkyl that is unsubstituted or substituted by alkoxy, alkoxycarbonyl, hydroxy, hydroxycarbonyl, halogen or by aryl; alkenyl that is unsubstituted or substituted by alkoxy, alkoxycarbonyl, hydroxycarbonyl, halogen or by aryl; or alkynyl that is unsubstituted or substituted by alkoxy, alkoxycarbonyl, hydroxycarbonyl, halogen or by aryl;
R₃ and R₄ are each independently of the other hydrogen or alkyl;
or R₂ and R₃ together are -(CH₂)_{q}- wherein q is an integer from 3 to 5, or R₂ and R₃ together are a divalent radical of formula (III) 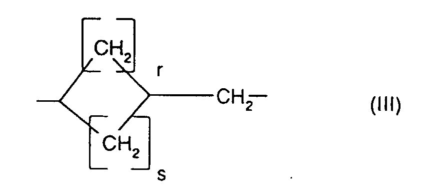 wherein r and s are each independently of the other an integer from 1 to 3; or
R₃ and R₄ together are -(CH₂)ₚ- wherein p is an integer from 3 to 5;
n and m are each independently of the other an integer from 10 to 100 000; and the sum of n and m is likewise an integer from 10 to 100 000, with the proviso that at least 20 % of all the segments each have at least one unsaturated carbon-carbon bond;
with a polysiloxane (b) that contains hydrosilane groups of formula (IV) 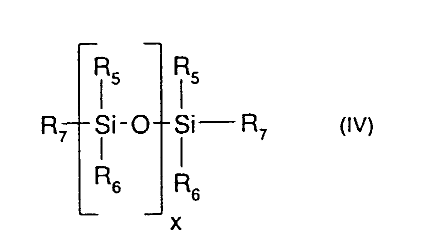 wherein the index x is from 1 to 1000; and the radicals R₅, R₆ and R₇ are each independently of the others hydrogen, alkyl, alkoxy, phenyl or fluorine-containing lower alkyl having up to 8 carbon atoms, with the proviso that in a compound of formula (IV) at least two of the radicals R₅, R₆ and R₇ and a maximum of 20 % of all the radicals R₅, R₆ and R₇ are hydrogen;
in such a mass ratio that the crosslinked product resulting therefrom has a polysiloxane content of from 30 to 80 % by weight.

2. An ophthalmic moulding made from a graft polymer obtainable by reacting a crosslinked product according to claim 1 with a hydrophilic or hydrophobic vinyl monomer.

3. An ophthalmic moulding according to either claim 1 or claim 2, wherein the unsaturated polymer (a) contains repeating units selected from units of formulae (I) and (II) wherein R₁, R₃ and R₄ are hydrogen and R₂ is lower alkenyl or halo-substituted lower alkenyl having up to 8 carbon atoms.

4. An ophthalmic moulding according to either claim 1 or claim 2, wherein the unsaturated polymer (a) contains repeating units selected from units of formulae (I) and (II) wherein R₁, R₃ and R₄ are hydrogen and R₂ is lower alkenyl having up to 4 carbon atoms.

5. An ophthalmic moulding according to either claim 1 or claim 2, wherein the unsaturated polymer (a) contains repeating units of formula (I) wherein R₁, R₃ and R₄ are hydrogen and R₂ is lower alkenyl having up to 4 carbon atoms.

6. An ophthalmic moulding according to either claim 1 or claim 2, wherein the unsaturated polymer (a) contains repeating units of formula (II) wherein R₁ is tri-lower alkylsilyl and R₂ is lower alkyl.

7. An ophthalmic moulding according to either claim 1 or claim 2, wherein the unsaturated polymer (a) contains alternating repeating units of formulae (I) and (II) wherein R₁, R₃ and R₄ are hydrogen and R₂ is lower alkyl or lower alkenyl having up to 4 carbon atoms.

8. An ophthalmic moulding according to either claim 1 or claim 2, wherein the unsaturated polymer (a) is selected from syndiotactic poly-1,2-butadiene, poly-1,4-butadiene and polyisoprene.

9. An ophthalmic moulding according to any one of claims 1 to 8, wherein the polysiloxane (b) is a compound of formula (IV) in which exactly two of the radicals R₅, R₆ and R₇ are hydrogen.

10. An ophthalmic moulding according to claim 9, wherein the two radicals R₇ are hydrogen.

11. An ophthalmic moulding according to any one of claims 1 to 10, wherein the polysiloxane (b) is a compound of formula (IV), two silicon-hydrogen bonds are in terminal positions and the radicals R₅ and R₆ are lower alkyl, fluorine-containing lower alkyl or phenyl.

12. An ophthalmic moulding according to claim 11, wherein the radicals R₅ and R₆ are lower alkyl having up to 8 carbon atoms and are especially methyl.

13. An ophthalmic moulding according to any one of claims 1 to 12, wherein x is from 1 to 500, especially from 1 to 200 or from 1 to 100 and more especially from 2 to 85.

14. An ophthalmic moulding according to either claim 1 or claim 2, wherein the unsaturated polymer (a) is selected from the group of a polymeric conjugated, aliphatic or alicyclic, conjugated diene substituted by halogen or by lower alkyl; a polymer of an alkyne or dialkyne that is unsubstituted or substituted by lower alkyl or by trimethylsilyl; a copolymer of a conjugated diene with a hydrophilic or hydrophobic vinyl monomer; or a partially hydrogenated derivative of the said compounds.

15. An ophthalmic moulding according to any one of claims 2 to 14, wherein the hydrophobic vinyl monomer is selected from C₁-C₁₈alkyl and C₃-C₁₈cycloalkyl acrylate and methacrylate, C₃-C₁₈alkyl-acrylamide and -methacrylamide, acrylonitrile, methacrylonitrile, vinyl-C₁-C₁₈alkanoate, C₂-C₁₈alkene, C₂-C₁₈haloalkene, styrene, lower alkylstyrene, lower alkyl vinyl ether, C₂-C₁₀perfluoroalkyl acrylate and methacrylate and corresponding partially fluorinated acrylate and methacrylate, C₃-C₁₂perfluoroalkylethylthiocarbonylaminoethyl acrylate and methacrylate, acryloxy- and methacryloxyalkylsiloxane, N-vinylcarbazole and C₁-C₁₂alkyl esters of maleic acid, fumaric acid, itaconic acid and mesaconic acid.

16. An ophthalmic moulding according to any one of claims 2 to 14, wherein the hydrophilic vinyl monomer is selected from hydroxy-substituted lower alkyl acrylate and methacrylate, acrylamide, methacrylamide, lower alkyl-acrylamide and -methacrylamide, ethoxylated acrylate and methacrylate, hydroxy-substituted lower alkyl-acrylamide and -methacrylamide, hydroxy-substituted lower alkyl vinyl ether, sodium vinylsulfonate, sodium styrenesulfonate, 2-acrylamido-2-methylpropanesulfonic acid, N-vinylpyrrole, N-vinyl-2-pyrrolidone, 2- and 4-vinylpyridine, vinylically unsaturated carboxylic acids having a total of from 3 to 5 carbon atoms, amino-lower alkyl, mono- lower alkylamino-lower alkyl and di-lower alkylamino-lower alkyl acrylate and methacrylate and allyl alcohol.

17. An ophthalmic moulding according to any one of claims 1 to 16, which is a contact lens, a corneal implant or an artificial cornea.

18. The use of a polymer according to any one of claims 1 to 16 in the production of contact lenses.

19. The use of a polymer according to any one of claims 1 to 16 for the coating of polymer substrates for ophthalmic use.

20. The use of a polymer according to any one of claims 1 to 16 as a corneal implant or artificial cornea.

## Revendications

1. Un moulage ophtalmique obtenu par réaction d'un polymère insaturé (a), qui contient des unités récurrentes qui sont choisies parmi des unités de formule (I) et (II) 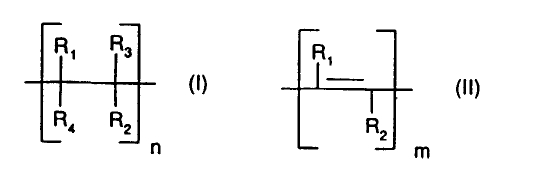 où
R₁ signifie l'hydrogène, un groupe alkyle ou trialkyl-silyle;
R₂ signifie un groupe alkyle qui est non substitué ou substitué par un groupe alcoxy, alcoxycarbonyle, hydroxy, hydroxycarbonyle, un halogène ou par un groupe aryle; un groupe alcényle qui est non substitué ou substitué par un groupe alcoxy, alcoxycarbonyle, hydroxycarbonyle, un halogène ou par un groupe aryle; ou bien un groupe alcynyle qui est non substitué ou substitué par un groupe alcoxy, alcoxycarbonyle, hydroxycarbonyle, un halogène ou par un groupe aryle;
R₃ et R₄ signifient indépendamment l'un de l'autre l'hydrogène ou un groupe alkyle;
ou bien R₂ et R₃ signifient ensemble -(CH₂)_{q}-, q signifiant un nombre entier de 3 à 5, ou bien R₂ et R₃ signifient ensemble un reste bivalent de formule (III), 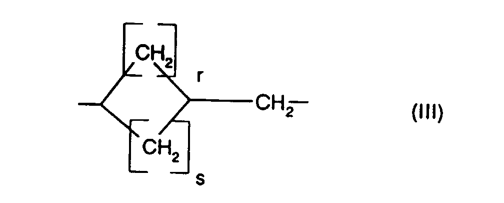 où r et s signifient indépendamment l'un de l'autre un nombre entier de 1 à 3; ou bien
R₃ et R₄ signifient ensemble -(CH₂)ₚ-, p signifiant un nombre entier de 3 à 5; n et m signifient indépendamment l'un de l'autre un nombre entier de 10 à 100 000; et la somme de n et m signifie également un nombre entier de 10 à 100 000, au moins 20% de tous les segments devant présenter chacun au moins une liaison carbone-carbone insaturée;
et d'un polysiloxane (b) contenant des groupes hydrosilane de formule (IV) 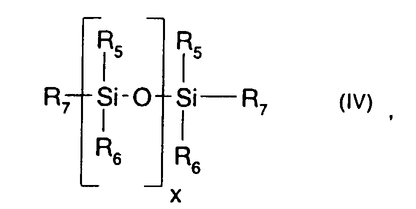 où l'indice x signifie de 1 à 1000; et les restes, R₅, R₆ et R₇ signifient indépendamment les uns des autres l'hydrogène, un groupe alkyle, alcoxy, phényle ou alkyle inférieur contenant du fluor ayant jusqu'à 8 atomes de carbone, dans un composé de formule (IV) au moins deux des restes R₅ , R₆ et R₇ et au plus 20% de tous les restes R₅, R₆ et R₇ devant signifier l'hydrogène;
dans un rapport de masse tel que le produit de réticulation ait une teneur en polysiloxane de 30 à 80% en poids.

2. Un moulage ophtalmique en polymère greffé qui est obtenu par réaction d'un produit de réticulation selon la revendication 1 avec un monomère vinylique hydrophile ou hydrophobe.

3. Un moulage ophtalmique selon la revendication 1 ou 2, dans lequel le polymère insaturé (a) contient des unités récurrentes qui sont choisies parmi des unités de formule (I) et (II), où R₁, R₃ et R₄ signifient l'hydrogène et R₂ signifie un groupe alcényle inférieur ou un groupe alcényle inférieur substitué par un halogène ayant jusqu'à 8 atomes de carbone.

4. Un moulage ophtalmique selon la revendication 1 ou 2, dans lequel le polymère insaturé (a) contient des unités récurrentes qui sont choisies parmi des unités de formule (I) et (II), où R₁, R₃ et R₄ signifient l'hydrogène et R₂ signifie un groupe alcényle inférieur ayant jusqu'à 4 atomes de carbone.

5. Un moulage ophtalmique selon la revendication 1 ou 2, dans lequel le polymère insaturé (a) contient des unités récurrentes de formule (I), où R₁, R₃ et R₄ signifient l'hydrogène et R₂ signifie un groupe alcényle inférieur ayant jusqu'à 4 atomes de carbone.

6. Un moulage ophtalmique selon la revendication 1 ou 2, dans lequel le polymère insaturé (a) contient des unités récurrentes de formule (Il), où R₁ signifie un groupe tri-alkyl inférieur-silyle et R₂ signifie un groupe alkyle inférieur.

7. Un moulage ophtalmique selon la revendication 1 ou 2, dans lequel le polymère insaturé (a) contient des unités récurrentes alternées de formule (I) et (Il), où R₁, R₃ et R₄ signifient l'hydrogène et R₂ signifie un groupe alkyle inférieur ou alcényle inférieur ayant jusqu'à 4 atomes de carbone.

8. Un moulage ophtalmique selon la revendication 1 ou 2, dans lequel le polymère insaturé (a) est choisi parmi le poly-1,2-butadiène, le poly-1,4-butadiène et le polyisoprène syndiotactiques.

9. Un moulage ophtalmique selon l'une des revendications 1 à 8, dans lequel le polysiloxane (b) signifie un composé de formule (IV) où exactement deux des restes R₅, R₆ et R₇ signifient l'hydrogène.

10. Un moulage ophtalmique selon la revendication 9, dans lequel les deux restes R₇ signifient l'hydrogène.

11. Un moulage ophtalmique selon l'une des revendications 1 à 10, dans lequel le polysiloxane (b) signifie un composé de formule (IV), deux liaisons silicium-hydrogène sont en position terminale et les restes R₅ et R₆ signifient un groupe alkyle inférieur, alkyle inférieur contenant du fluor ou phényle.

12. Un moulage ophtalmique selon la revendication 11, dans lequel les restes R₅ et R₆ signifient un groupe alkyle inférieur ayant jusqu'à 8 atomes de carbone et signifient en particulier un groupe méthyle.

13. Un moulage ophtalmique selon l'une des revendications 1 à 12, dans lequel x signifie de 1 à 500, en particulier de 1 à 200 ou de 1 à 100 et en particulier de préférence de 2 à 85.

14. Un moulage ophtalmique selon la revendication 1 ou 2, dans lequel le polymère insaturé (a) est choisi parmi le groupe d'un diène polymère conjugué, aliphatique ou alicyclique et conjugué qui est substitué par un halogène ou un groupe alkyle inférieur; un polymère d'une alcyne ou d'une dialcyne qui est non substitué ou substitué par un groupe alkyle inférieur ou triméthylsilyle; un copolymère d'un diène conjugué avec un monomère vinylique hydrophile ou hydrophobe; ou bien un dérivé partiellement hydrogéné des composés indiqués.

15. Un moulage ophtalmique selon l'une des revendications 2 à 14, dans lequel le monomère vinylique hydrophobe est choisi parmi l'acrylate et le méthacrylate de C₁-C₁₈-alkyle et C₃-C₁₈-cycloalkyle, le C₃-C₁₈-alkylacrylamide et -méthacrylamide, l'acrylonitrile, le méthacrylonitrile, le vinyl-C₁-C₁₈-alcanoate, le C₂-C₁₈-alcène, le C₂-C₁₈-haloalcène, le styrène, l'alkyl inférieur-styrène, l'alkyl inférieur-vinyléther, l'acrylate et le méthacrylate de C₂-C₁₀-perfluoroalkyle ou l'acrylate et le méthacrylate partiellement fluorés correspondants, l'acrylate et le méthacrylate de C₃-C₁₂-perfluoroalkyl-éthyl-thiocarbonylamino-éthyle, l'acryloxy- et le méthacryloxyalkylsiloxane, le N-vinylcarbazole et l'ester C₁-C₁₂-alkylique de l'acide maléinique, de l'acide fumarique, de l'acide itaconique et de l'acide mésaconique.

16. Un moulage ophtalmique selon l'une des revendications 2 à 14, dans lequel le monomère vinylique hydrophile est choisi parmi l'acrylate et le méthacrylate d'alkyle inférieur substitué par un groupe hydroxy, l'acrylamide, le méthacrylamide, l'alkyl inférieur-acrylamide et méthacrylamide, l'acrylate et le méthacrylate éthoxylés, l'alkyl inférieur-acrylamide et méthacrylamide substitués par un groupe hydroxy, l'alkyl inférieur-vinyléther substitué par un groupe hydroxy, le vinylsulfonate de sodium, le styrènesulfonate de sodium, l'acide 2-acrylamido-2-méthylpropanesulfonique, le N-vinylpyrrole, la N-vinyl-2-pyrrolidone, la 2- et 4-vinylpyridine, les acides carboxyliques à insaturation vinylique ayant un total de 3 à 5 atomes de carbone, l'acrylate et le méthacrylate d'amino-alkyle inférieur, de mono-alkyl inférieur amino-alkyle inférieur et de di-alkyl inférieur-amino-alkyle inférieur et l'alcool allylique etc...

17. Un moulage ophtalmique selon l'une des revendications 1 à 16,
caractérisé en ce qu'il s'agit d'une lentille de contact, d'un implant de cornée ou d'une cornée artificielle.

18. Utilisation d'un polymère selon l'une des revendications 1 à 16, pour la préparation de lentilles de contact.

19. Utilisation d'un polymère selon l'une des revendications 1 à 16, pour l'enrobage de substrats polymères utilisables en ophtalmologie.

20. Utilisation d'un polymère selon l'une des revendications 1 à 16, comme implant de cornée ou cornée artificielle.
